(19) Europäisches Patentamt / European Patent Office / Office européen des brevets

(11) **EP 2 559 375 A1**

(12) **EUROPEAN PATENT APPLICATION**

(43) Date of publication:
20.02.2013 Bulletin 2013/08

(51) Int Cl.:
*A61B 5/01* (2006.01)    *G01K 11/22* (2006.01)
*A61N 7/02* (2006.01)    *A61B 8/00* (2006.01)

(21) Application number: **12180845.5**

(22) Date of filing: **17.08.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **19.08.2011 KR 20110083051**

(71) Applicant: **Samsung Electronics Co., Ltd.**
**Suwon-si, Gyeonggi-do, 443-742 (KR)**

(72) Inventors:
• **Cho, Joon-kee**
**Gyeonggi-do (KR)**
• **Choi, Ki-wan**
**Gyeonggi-do (KR)**
• **Kong, Dong-geon**
**Gyeonggi-do (KR)**

(74) Representative: **Grootscholten, Johannes A.M. et al**
**Arnold & Siedsma**
**Sweelinckplein 1**
**2517 GK Den Haag (NL)**

(54) **Method and device for monitoring temperature of treatment site by using ultrasound, and system for treatment and diagnosis using ultrasound**

(57)     According to a method and device for monitoring temperature by using ultrasound, an echo signal of diagnostic ultrasound irradiated to a treatment site is acquired, candidate temperature images are generated from the echo signal using different temperature determining methods, and the generated candidate temperature images are merged, resulting in generating a final temperature image.

FIG. 3A

**Description**

BACKGROUND

1. Field

One or more embodiments of the present disclosure relate to a method and a device for monitoring a temperature of a treatment site using ultrasound, and a system for treatment and diagnosis using ultrasound.

2. Description of the Related Art

With the development of medical science, topical treatment of tumors has been developed in relation to invasive surgery, such as laparotomy, minimal-invasive surgery, etc. In addition, as non-invasive surgery, a gamma knife, a cyber knife, a high intensity focused ultrasound (HIFU) knife, and the like have been developed. In particular, the latest HIFU knife has been widely used by using ultrasound as a safe and eco-friendly treatment.
Treatments using the HIFU knife include a surgery for removing and treating tumors by causing focal destruction or necrosis of tumor tissue as a result of the irradiation of HIFU to the tumor.

SUMMARY

One or more embodiments of the present disclosure relate to a method and a device for monitoring a temperature of a treatment site, such as a tumor and peripheral areas thereof in real time while the treatment site such as the tumor is being treated.
One or more embodiments of the present disclosure relate to a computer-readable recording medium storing a computer-readable program for executing the method.
One or more embodiments of the present disclosure relate to a system for treatment and temperature monitoring of a treatment site, such as a tumor, using ultrasound.
Additional aspects will be set forth in part in the description which follows and, in part, will be apparent from the description, or may be learned by practice of the presented embodiments.
According to an embodiment, a method of monitoring temperature by using ultrasound includes: acquiring an echo signal of diagnostic ultrasound that is irradiated to a treatment site; generating candidate temperature images from the echo signal by using different temperature determining methods so that the candidate temperature images correspond to each of the temperature determining methods; merging the generated candidate temperature images by applying different accuracies of the temperature determining methods to the generated candidate temperature images; and generating a final temperature image based on the merging.
According to another embodiment, there is provided a computer-readable recording medium storing a computer-readable program for executing the method of monitoring temperature.
According to another embodiment, a device for monitoring temperature by using ultrasound includes: an acquisition unit that acquires an echo signal of diagnostic ultrasound irradiated to a treatment site; a candidate temperature image generating unit that generates candidate temperature images from the acquired echo signal by using different temperature determining methods so that the candidate temperature images correspond to each of the temperature determining methods; a merging unit that merges the generated candidate temperature images by respectively applying different accuracies of the temperature determining methods to the generated candidate temperature images; and a final temperature image generating unit that generates a final temperature image based on the merged results.
According to another embodiment, a system for treatment and diagnosis using ultrasound includes: a therapeutic ultrasound device that irradiates therapeutic ultrasound to a treatment site; a diagnostic ultrasound device that irradiates diagnostic ultrasound to the treatment site and receives an echo signal of the diagnostic ultrasound; a temperature monitoring device that acquires the received echo signal, generates candidate temperature images from the echo signal by using different temperature determining methods so that the candidate temperature images correspond to each of the temperature determining methods, merging the generated candidate temperature images by applying different accuracies of the temperature determining methods to the generated candidate temperature images, and generating a final temperature image based on the merging; and a display device that displays at least one selected from the candidate temperature images and the final temperature image.

BRIEF DESCRIPTION OF THE DRAWINGS

The patent or application file contains at least one drawing executed in color. Copies of this patent or patent application publication with color drawing(s) will be provided by the Office upon request and payment of the necessary fee. These and/or other aspects will become apparent and more readily appreciated from the following description of the embodiments, taken in conjunction with the accompanying drawings of which:

FIG. 1 is a schematic diagram of a system for treatment and diagnosis using ultrasound, according to an embodiment of the present disclosure;

FIG. 2 is a block diagram of a temperature monitoring device according to an embodiment of the present disclosure;

FIGS. 3A and 3B are diagrams for describing methods of determining a temperature;

FIG. 4 shows candidate temperature images according to an embodiment of the present disclosure;

FIG. 5 shows positions of focuses in candidate temperature images and a final temperature image according to an embodiment of the present disclosure;

FIG. 6 shows a process of generating candidate error images performed by a candidate error image generating unit;

FIG. 7 shows candidate error images according to an embodiment of the present disclosure;

FIG. 8 shows a final temperature image and a final error image according to an embodiment of the present disclosure;

FIG. 9 is a graph illustrating a monitored temperature according to an embodiment of the present disclosure compared with a monitored temperature according to general methods; and

FIG. 10 is a flowchart of a method of monitoring temperature by using ultrasound according to an embodiment of the present disclosure.

DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description.

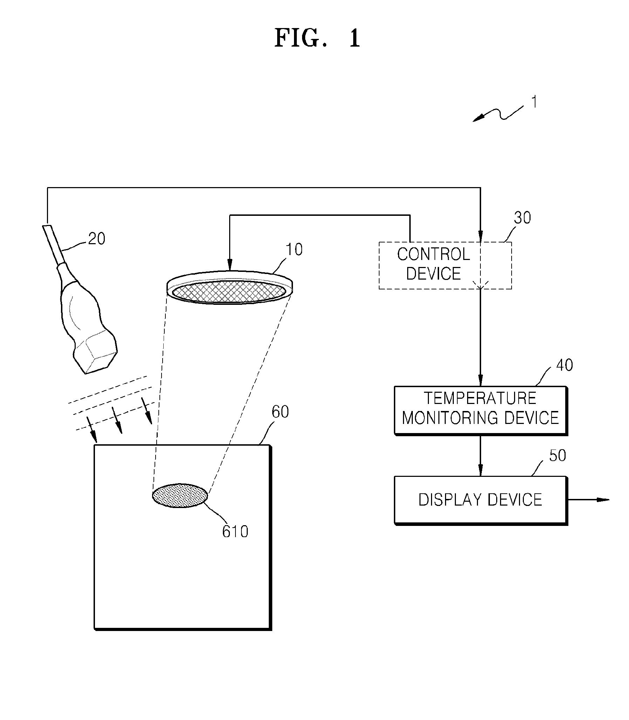

FIG. 1 is a schematic diagram of a system for treatment and diagnosis 1 using ultrasound, according to an embodiment of the present disclosure. Referring to FIG. 1, the system for treatment and diagnosis 1 using ultrasound according to an embodiment of the present disclosure may include, for example, a therapeutic ultrasound device 10, a diagnostic ultrasound device 20, a control device 30, a temperature monitoring device 40, and a display device 50.

Only elements of the system for treatment and diagnosis 1 using ultrasound that are particularly associated with this embodiment are shown in FIG. 1. However, it will be understood by those of ordinary skill in the art that general-use elements may also be included.

The system for treatment and diagnosis 1 using ultrasound is a system involving the treatment of select tissue such as a tumor 60 of a patient by irradiating therapeutic ultrasound to a treatment site 610 of the tumor 60 and monitoring results of the treatment, such as a temperature of the treatment site 610, using diagnostic ultrasound. That is, the system for treatment and diagnosis 1 using ultrasound is a medical system involving necrosing of the tumor 60 of a patient using ultrasound and diagnosing the patient by monitoring the results in real time.

More particularly, according to the system for treatment and diagnosis 1 using ultrasound, if the tumor 60 is found in a patient, therapeutic ultrasound may be irradiated to the treatment site 610 of the tumor 60 using the therapeutic ultrasound device 10 to form a lesion, and ultrasound images of the treatment site 610 may be acquired using the diagnostic ultrasound device 20, resulting in a diagnosis of whether the treatment is successful. Such a lesion is caused by focal destruction or necrosis of tissues of the treatment site 610.

Furthermore, according to the system for treatment and diagnosis 1 using ultrasound according to the current embodiment, a temperature variation of the treatment site 610 may also be monitored using the diagnostic ultrasound device 20.

In therapeutic ultrasound treatment, such as high intensity focused ultrasound (HIFU), when the HIFU arrives at a portion of the tumor 60, a temperature of the portion of the tumor 60 is instantly increased to 70°C or more due to thermal energy from the HIFU. It is known that tissues are theoretically destroyed when exposed to a temperature of about 60°C for 100 msec or more. Coagulative necrosis occurs in tissues and vessels around the tumor 60 at such a high temperature.

According to the current embodiment, the temperature variation of the treatment site 610 may be monitored in real time. As a result, ultrasound treatment may be efficiently performed by accurately determining whether further treatment is required or whether the treatment has been successful.

Referring to FIG. 1, operations of elements of the system for treatment and diagnosis 1 using ultrasound will respectively be described.

The therapeutic ultrasound device 10 treats the patient by irradiating therapeutic ultrasound to the treatment site 610 of the tumor 60 so as to necrose tissues of the treatment site 610. In other words, the therapeutic ultrasound device 10 generates therapeutic ultrasound and irradiates the therapeutic ultrasound to select tissue of the patient such as topical tissue.

The therapeutic ultrasound according to the current embodiment may be administered using a HIFU device. That is, the therapeutic ultrasound device 10 according the current embodiment is a device that irradiates tissue using HIFU, widely known as therapeutic ultrasound. The HIFU device will be apparent to one of ordinary skill in the art, and thus detailed descriptions thereof will be omitted herein. However, it will be apparent to one of ordinary skill in the art that the therapeutic ultrasound device 10 is not limited to the device irradiating HIFU, and any device irradiating focused ultrasound similar to the HIFU may also be used as the therapeutic ultrasound device 10.

The diagnostic ultrasound device 20, which is also referred to as a diagnostic probe, irradiates diagnostic ultrasound to the treatment site 610 and receives an echo signal that is a reflected diagnostic ultrasound. In this regard, the diagnostic ultrasound device 20 generally irradiates diagnostic ultrasound to the treatment site 610 and receives the reflected echo signal in order to generate an ultrasound diagnostic image of the treatment site 610. A process of generally generating ultrasound images using the reflected echo signal will be apparent to one of ordinary skill in the art, and thus detailed descriptions thereof will be omitted herein.

Although the therapeutic ultrasound device 10 and the diagnostic ultrasound device 20 are independent devices according to the current embodiment, the present disclosure is not limited thereto. The therapeutic ultrasound device 10 and the diagnostic ultrasound device 20 may be realized as separate modules in one device or as one device. That is, the therapeutic ultrasound device 10 and the diagnostic ultrasound device 20 are not limited to any one configuration.

According to the current embodiment, the echo signal received by the diagnostic ultrasound device 20 is also used to monitor the treatment site 610 and a temperature variation at and around the treatment site 610. In other words, the echo signal may be used not only to generate ultrasound diagnostic images but also to monitor the temperature variation of the treatment site 610.

A change in backscattered energy (CBE) method has been used as a method of determining a temperature or for monitoring temperature using ultrasound. Briefly, the CBE method is a method of measuring a temperature variation by detecting an amplitude variation of a wave in an echo signal of ultrasound. According to the CBE method, when ultrasound passes through tissues having a high temperature, a tail artifact phenomenon, in which temperature errors rapidly increase at the tissues, occurs. Thus, errors increase and temperature distribution cannot be detected in detail due to low spatial resolution according to the CBE method.

An echo-shift (ES) method has also been used as a method of determining a temperature. Briefly, the ES method is a method of measuring a temperature variation by detecting a speed variation of a wave of an echo signal of diagnostic ultrasound. High-intensity focused ultrasound for example for tissue or tumor treatment causes temperature changes in treated tissue or tumors which results in time shifts in the echoes that traverse the heated tissue. These time shifts are caused by thermally induced changes in the distribution of the velocity of sound through a tissue or a tumor and by thermal expansion within the tissue. According to the ES method, however, while a low temperature range of about 36 to 43°C may be relatively accurately measured, a high temperature range over 43°C cannot be accurately measured.

That is, an accurately measurable temperature range is limited, and a side effect, such as a tail artifact, occurs according to known methods of monitoring temperature using ultrasound. Thus, a wide temperature range of the treatment site 610 cannot be monitored in real time during ultrasound treatment.

However, the temperature monitoring device 40 according to the current embodiment determines the temperature variation from the echo signal of the diagnostic ultrasound by merging various temperature determining methods, whereby temperatures may be accurately monitored over a relatively wide temperature range. Hereinafter, operations and functions of the temperature monitoring device 40 according to the current embodiment will be described in more detail.

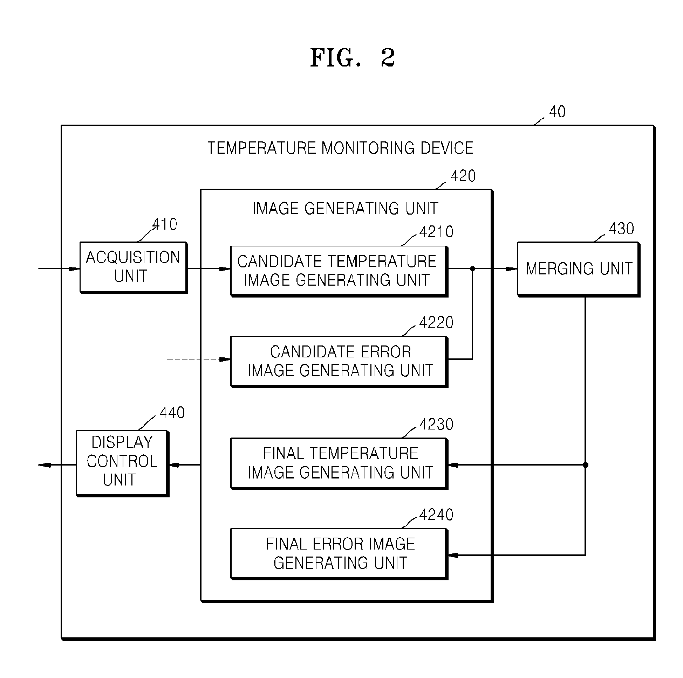

FIG. 2 is a block diagram of a temperature monitoring device 40 according to an embodiment of the present disclosure. Referring to FIG. 2, the temperature monitoring device 40 may include, for example, an acquisition unit 410, an image generating unit 420, a merging unit 430, and a display control unit 440.

In one or more embodiments, the temperature monitoring device 40 may be a processor. That is, the temperature monitoring device 40 may include an array of a plurality of logic gates or a combination of a universal microprocessor and memories in which programs that may be implemented in the universal microprocessor are stored. Furthermore, it will be understood by those of ordinary skill in the art that the temperature monitoring device 40 may be implemented by another form of hardware.

Meanwhile, only elements of the system for treatment and diagnosis 1 using ultrasound that are particularly associated with this embodiment are shown in FIG. 2. However, it will be understood by those of ordinary skill in the art that general-use elements may also be included.

The acquisition unit 410 acquires an echo signal from the diagnostic ultrasound that is irradiated by the diagnostic ultrasound device 20 (FIG. 1) and reflected by tissues of a human body. In an embodiment, the echo signal may be a waveform signal.

The image generating unit 420 generates a temperature image or an error image of the treatment site 610 (FIG. 1) including the tumor 60 (FIG. 1) and peripheral areas thereof. In one or more embodiments, the temperature image may be an image that displays a temperature distribution of the treatment site 610 (FIG. 1) and peripheral areas thereof using different colors of different brightness. The error image may be an image that is mapped with pixels of the temperature image and displays errors of the temperature distribution using different colors of different brightness. The image generating unit 420 may include, for example, a candidate temperature image generating unit 4210, a candidate error image generating unit 4220, a final temperature image generating unit 4230, and a final error image generating unit 4240.

The candidate temperature image generating unit 4210 generates candidate temperature images from the echo signal acquired by the acquisition unit 410 using different temperature determining methods so as to correspond to each of the temperature determining methods.

The different temperature determining methods used in the candidate temperature image generating unit 4210 include methods of detecting a plurality of different types of waveform variations from the echo signal. In this regard, the different types of waveform variations include at least two selected from the group of waveform variations including echo time variation, amplitude variation, and shape variation of the echo signal. In one or more embodiments, the shape variation of the echo signal includes frequency variation or nonlinear variation of the echo signal. That is, according to an embodiment, the temperature is monitored by merging the plurality of temperature determining methods instead of using one temperature determining method.

In an embodiment, the method of determining a temperature using the waveform variation such as echo time variation of the echo signal may be similar to the above-mentioned ES method, and the method of determining temperature using the waveform variation such as an amplitude variation of the echo signal may be similar to the above-mentioned CBE method.

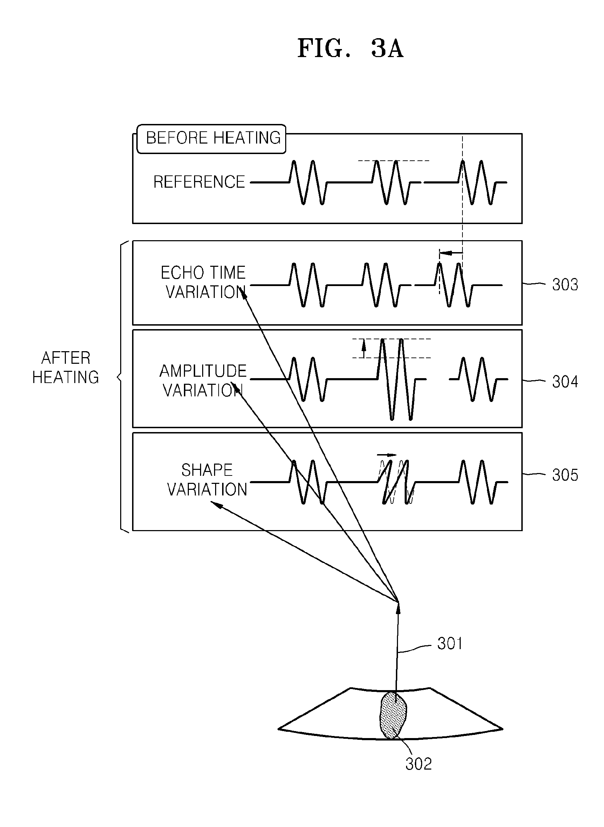

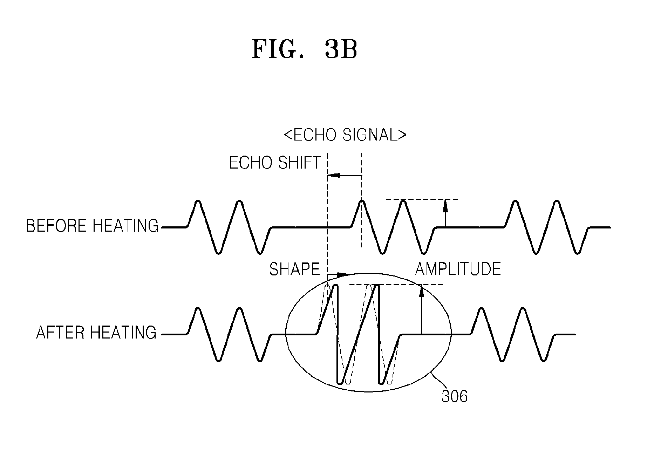

FIGS. 3A and 3B illustrate methods of determining temperature.

Referring to FIG. 3A, the candidate temperature image generating unit 4210 (FIG. 2) may detect at least two waveform variations selected from the group of waveform variations including echo time variation, amplitude variation, and shape variation of the echo signal 301.

First, a method of detecting echo time variation 303 of the echo signal 301 will be described.

After the therapeutic ultrasound is irradiated to the treatment site 302, the candidate temperature image generating unit 4210 (FIG. 2) may compare the echo signal 301 with a reference signal to detect the echo time variation 303 of the echo signal 301.

More particularly, the candidate temperature image generating unit 4210 (FIG. 2) may detect whether a delay occurs in the echo signal 301 after the comparison with the reference, and then may detect whether the echo signal 301 varies in the delay. Then, the candidate temperature image generating unit 4210 (FIG. 2) may determine a temperature by mapping the degree of delay or the degree of echo time variation with the reference information stored in advance.

In this regard, only 3 cycles of the waveform of the echo time variation 303 are shown. However, there may be more or less than 3 cycles of the waveform detected. Accordingly, the candidate temperature image generating unit 4210 (FIG. 2) may detect a temperature distribution of the treatment site 302 and peripheral area thereof by detecting the echo time variation 303 of each cycle. The candidate temperature image generating unit 4210 (FIG. 2) generates a candidate temperature image corresponding to the echo time variation 303 by using the detected temperature distribution based on the echo time variation 303.

Next, a method of detecting amplitude variation 304 of the echo signal 301 will be described.

After the therapeutic ultrasound is irradiated to the treatment site 302, the candidate temperature image generating unit 4210 (FIG. 2) may compare the echo signal 301 with a reference signal to detect the amplitude variation 304 of the echo signal 301.

More particularly, the candidate temperature image generating unit 4210 (FIG. 2) may compare the amplitude of the echo signal 301 with the reference signal, resulting in detecting variation. Then, the candidate temperature image generating unit 4210 (FIG. 2) maps the degree of the amplitude variation with a temperature of the reference information, resulting in the determining of a temperature.

In this regard, only 3 cycles of the waveform of the amplitude variation 304 are shown. However, there may be more or less than 3 cycles of the waveform detected. Accordingly, the candidate temperature image generating unit 4210 (FIG. 2) may detect temperature distribution of the treatment site 302 and peripheral areas thereof by detecting the amplitude variation 304 of each cycle. The candidate temperature image generating unit 4210 (FIG. 2) generates a candidate temperature image corresponding to the amplitude variation 304 by using the detected temperature distribution based on the amplitude variation 304.

Finally, a method of detecting shape variation 305 of the echo signal 301 will be described. This method is a frequency shift (FS) method. The FS method is based on the phenomenon, that frequency changes in harmonics are linear with temperature changes. Consequently, images can be constructed by detecting the frequency changes in harmonics prior to and after or during heating the tissue or tumor.

After the therapeutic ultrasound is irradiated to the treatment site 302, the candidate temperature image generating unit 4210 (FIG. 2) may compare the echo signal 301 with a reference signal to detect the shape variation 305 of the echo signal 301.

More particularly, the candidate temperature image generating unit 4210 (FIG. 2) may compare the echo signal 301 with the reference signal to detect a variation of frequency in the waveform of the echo signal 301, and may then detect whether the shape of the echo signal 301 is changed by the variation. Then, the candidate temperature image generating unit 4210 (FIG. 2) maps the degree of the shape variation with a temperature of the reference information, resulting in the determining of a temperature.

In this regard, only 3 cycles of the waveform of the shape variation 305 are shown. However, there may be more or less than 3 cycles of the waveform detected. Accordingly, the candidate temperature image generating unit 4210 (FIG. 2) may detect the temperature distribution of the treatment site 305 and peripheral areas thereof by detecting the shape variation 305 of each cycle. The candidate temperature image generating unit 4210 (FIG. 2) generates a candidate temperature image corresponding to the shape variation 305 by using the detected temperature distribution based on the shape variation 305.

FIG. 3B shows echo signals before the irradiation of the therapeutic ultrasound (before heating) and after the irradiation of the therapeutic ultrasound (after heating). The echo signal after the irradiation of the therapeutic ultrasound may include any of the echo shift, amplitude variation, and shape variation of the waveform 306, in which temperature variation of the treatment site 302 (FIG. 3A) is reflected. In other words, if the temperature is changed, at least one waveform variation is reflected in the echo signal. When each of the temperature determining methods is used, waveform variations of the echo signal may be respectively detected. Thus, a temperature image corresponding to each of the temperature determining methods may be detected.

That is, different types of waveform variations may be reflected in the echo signal while the temperature of the treatment site 302 (FIG. 3A) is changed. The candidate temperature image generating unit 4210 (FIG. 2) generates a plurality of candidate temperature images corresponding to each of the temperature determining methods by using different temperature determining methods.

3 types of temperature determining methods have been described with reference to FIGS. 3A and 3B. The candidate temperature image generating unit 4210 (FIG. 2) may generate candidate temperature images corresponding to each of the temperature determining methods by using at least two of the temperature determining methods.

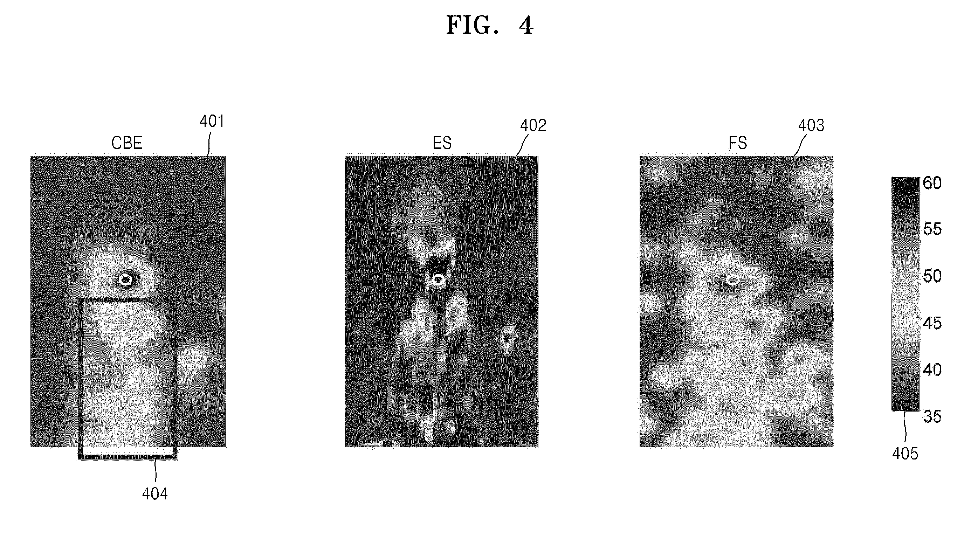

FIG. 4 shows candidate temperature images according to an embodiment of the present disclosure. Referring to FIG. 4, candidate temperature images 401, 402, and 403 corresponding to each of the temperature determining methods are shown. That is, FIG. 4 shows a CBE image 401 corresponding to the CBE method, an ES image 402 corresponding to the ES method, and a FS image 403 corresponding to the FS method. Such candidate temperature images 401, 402, and 403 may be generated by the candidate temperature image generating unit 4210 (FIG. 2). Furthermore, as described above, the candidate temperature image generating unit 4210 (FIG. 2) generates at least two of the candidate temperature images 401, 402, and 403 shown in FIG. 4.

The candidate temperature images 401, 402, and 403 are shown having various colors. In the color reference information 405, different colors are mapped according to their temperature. Thus, various colors of pixels of the candidate temperature images 401, 402, and 403 indicate different temperatures according to the color reference information 405. Although FIG. 4 shows different colors, the present disclosure is not limited thereto. The candidate temperature images 401, 402, and 403 may also be displayed as black and white images displayed using different brightness according to their temperature.

Pixels of each of the candidate temperature images 401, 402, and 403 display temperature information of a same site in the body corresponding to the pixels, such as treatment site 610 (FIG. 1). However, in each of the candidate temperature images 401, 402, and 403, although the temperature images are obtained from the same treatment site 610 (FIG. 1) and peripheral areas thereof, different temperature distributions may be detected. For example, in the CBE image 401, errors may increase due to a tail artifact phenomenon 404 as illustrated at FIG. 4.

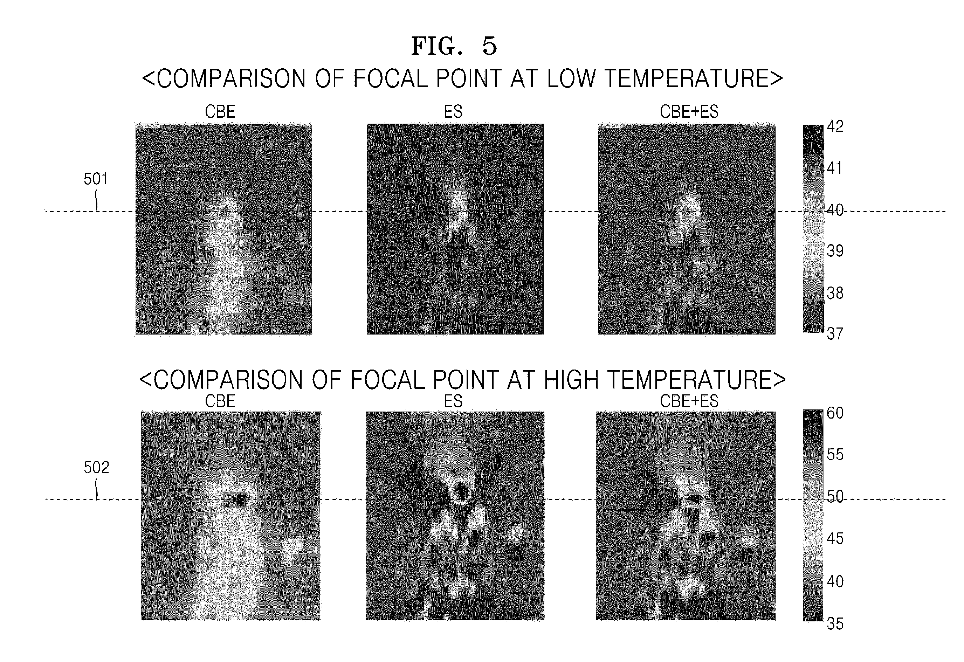

FIG. 5 shows positions of focuses in candidate temperature images and a final temperature image according to an embodiment of the present disclosure. In FIG. 5, positions of focuses indicate treatment sites to which the therapeutic ultrasound is irradiated.

FIG. 5 shows CBE images and ES images at a low temperature and a high temperature. FIG. 5 also shows a CBE + ES image (or Fusion image) which is a final temperature image generated by merging temperature information of the candidate temperature images. The final temperature image will now be described in more detail.

First, there is no difference in the positions of focuses 501 in the CBE image, the ES image, and the CBE + ES image located along the same line. However, when the temperature of the treatment site increases due to the ultrasound treatment, the position of a focus 502 is accurately reflected in the CBE image at high temperature, but the position of the focus 502 is not accurately reflected in the ES image at high temperature. However, the original position of the focus 502 is accurately reflected in the CBE + ES image (Fusion image) at high temperature. As a result, temperature of the treatment site may not be accurately reflected in each of the candidate temperature images such as the CBE image and the ES image in a predetermined temperature range. However, if the final temperature image such as the CBE + ES (Fusion image) image is used, the temperature distribution may be relatively accurately displayed compared with using only a single candidate temperature image.

Referring back to FIG. 2, the candidate error image generating unit 4220 calculates errors of the temperature information contained in each of the candidate temperature images based on the accuracy of each of the temperature determining methods. Then, the candidate error image generating unit 4220 generates candidate error images that show the calculated error of each of the candidate temperature images. In an embodiment, pixel values of each of the candidate error images may refer to an absolute or relative size or distribution of the errors.

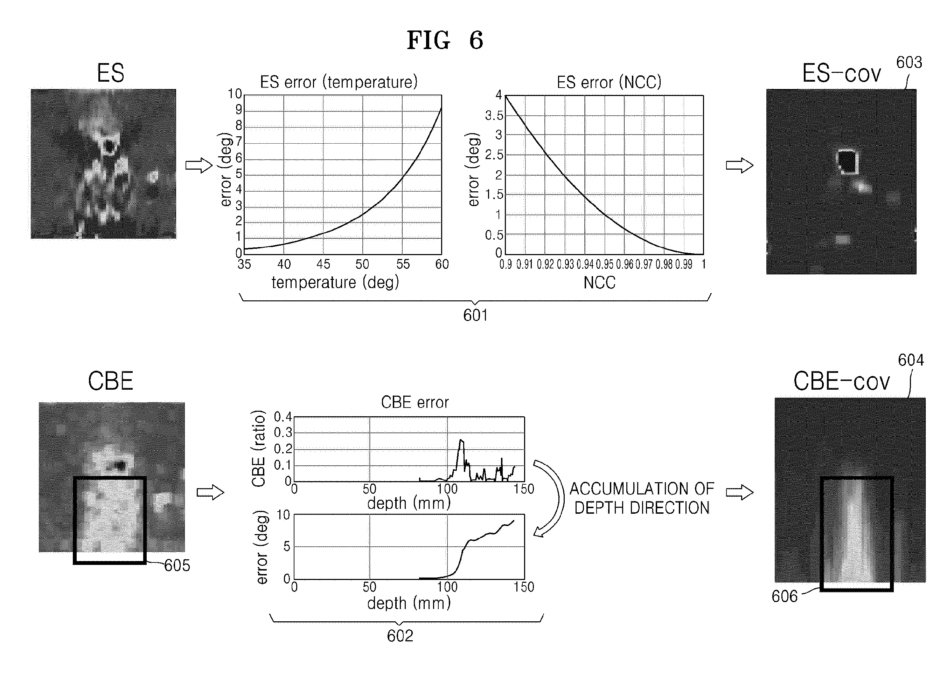

FIG. 6 shows a process of generating candidate error images performed by a candidate error image generating unit. Referring to FIG. 6, each of the temperature determining methods has a different accuracy in each temperature range.

First, a process of producing an ES-cov image 603 that is a candidate error image of the ES image will be described.

According to the ES method, errors increase as the temperature of the treatment site 610 increases (FIG. 1). This phenomenon may be apparent due to the relationship between the temperature and the ES error, and the relationship between the ES error and a normalized cross-correlation (NCC) value as shown in a graph 601. The graph 601 shows that as the NCC value approaches 1, accuracy of the ES method increases (that is, ES errors decrease), and that as the NCC value decreases, the accuracy of the ES method decreases (that is, ES errors increase) due to decorrelation. That is, the ES method is not as accurate at relatively high temperatures. Here, it will be apparent to one of ordinary skill in that art that the graph 601 is illustrative merely for descriptive convenience, and the graph 601 may vary according to errors obtained by results of quantitative experiments.

The candidate error image generating unit 4220 (FIG. 2) calculates errors of temperature information contained in the ES image that is a candidate temperature image using the accuracy of the ES method as shown in the graph 601 described above. Then, the candidate error image generating unit 4220 (FIG. 2) generates an ES-cov image 603 that is a candidate error image representing the calculated error.

A color value of each pixel of the ES-cov image 603 may correspond to a distribution of the errors. That is, the candidate error image such as the ES-cov image 603 may be displayed using different colors according to the size of the error. For example, in the ES-cov image 603, central red regions indicate regions having more errors, and other blue regions indicate regions having less errors.

Next, a process of producing a CBE-cov image 604 that is a candidate error image of the CBE image will be described.

As described above, according to the CBE method, errors occur due to the tail artifact phenomenon 605. This tail artifact phenomenon 605 occurs when the diagnostic ultrasound passes through the treatment site 610 (FIG. 1) at high temperature while proceeding in the depth direction. That is, referring to a graph 602, as the depth increases, errors increase by the tail artifact phenomenon 605. Here, it will be apparent to one of ordinary skill in that art that the graph 602 is illustrative merely for descriptive convenience, and the graph 602 may vary according to errors obtained by results of quantitative experiments.

The candidate error image generating unit 4220 (FIG. 2) calculates errors of temperature information contained in the CBE image that is a candidate temperature image using the accuracy of the CBE method as shown in the graph 602 described above. Then, the candidate error image generating unit 4220 (FIG. 2) generates a CBE-cov image 604 that is a candidate error image representing the calculated errors.

As in the ES-cov image 603, a color value of each pixel of the CBE-cov image 604 may correspond to a distribution of the errors. That is, the candidate error image such as the CBE-cov image 604 may be displayed using different colors according to the size of the error. For example, in the CBE-cov image 604, CBE error regions 606 caused by the tail artifact phenomenon 605 are displayed with a color different from the other regions.

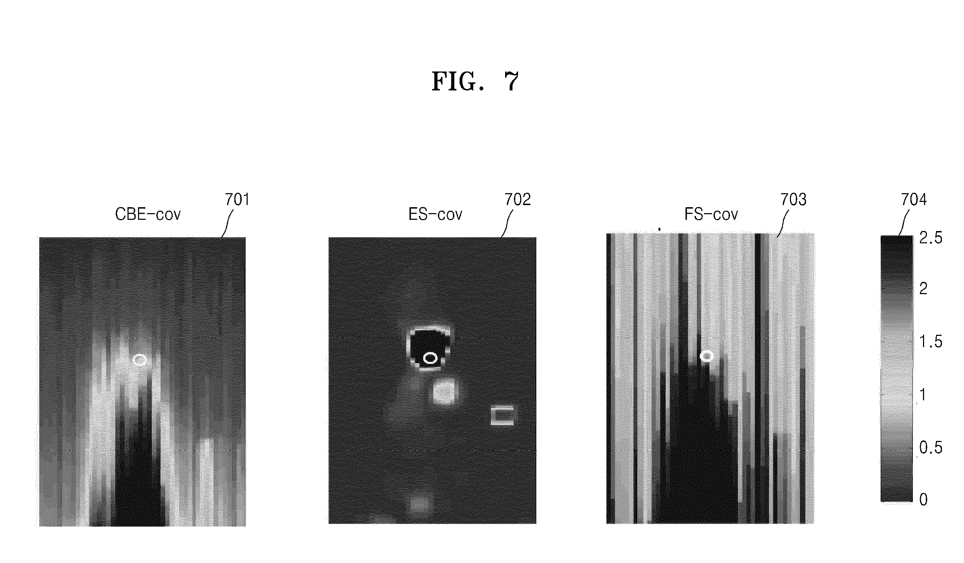

FIG. 7 shows candidate error images according to an embodiment of the present disclosure. FIG. 7 shows a CBE-cov image 701, an ES-cov image 702, and a FS-cov image 703 which include candidate error images.

Colors of pixels of the candidate error images correspond to colors of the color reference information 704. Color values of the color reference information 704 indicate a distribution and size of errors. Accordingly, the candidate error images are represented by colors corresponding to the distribution of errors and size of errors in each pixel according to the color reference information 704. However, it will be apparent to one of ordinary skill in that art that the reference of color values of the color reference information 704 may vary according to a use environment.

Referring back to FIG. 2, as described with reference to FIG. 6 above, the candidate error image generating unit 4220 (FIG. 2) generates candidate error images respectively corresponding to candidate temperature images generated by the candidate temperature image generating unit 4210 (FIG. 2). That is, according to the current embodiment, the candidate error image generating unit 4220 (FIG. 2) may generate at least two candidate error images selected from the group including the ES-cov image, the CBE-cov image, and the FS-cov image.

The merging unit 430 may merge the candidate temperature images by applying different accuracies of the temperature determining methods to the candidate temperature images generated by the candidate temperature image generating unit 4210.

In more detail, the merging unit 430 may merge the candidate temperature images by calculating final temperature information (or Fusion temperature information) using temperature information contained in each of the candidate temperature images and error information contained in each of the candidate error images. That is, the merging unit 430 merges different temperature information detected by each of the temperature determining methods, resulting in generating the final temperature information.

Furthermore, the merging unit 430 may further merge the error information contained in each of the candidate error images generated by the candidate error image generating unit 4220, resulting in the generating of final error information.

That is, the merging unit 430 calculates the final temperature information by merging the candidate temperature images, and calculates the final error information by merging the candidate error images. In this regard, the merging unit 430 merges temperature information represented by pixels respectively corresponding to the candidate temperature images. In addition, the merging unit 430 merges error information represented by pixels respectively corresponding to the candidate error images. This is because pixels corresponding to the candidate temperature images and candidate error images indicate the same position in the human body.

The merging unit 430 may merge the candidate temperature images by respectively allocating weights that correspond to the accuracies of the temperature determining methods to the temperature information contained in each of the candidate temperature images. For example, a relatively large weight may be allocated to temperature information of a first temperature determining method that is determined to be accurate over a given range, while a relatively small weight may be allocated to temperature information of a second temperature determining method that is determined to be relatively inaccurate over the given range.

For example, when the merging unit 430 merges the candidate temperature images of the CBE image and the ES image, the merging unit 430 may perform the merging process using Equations 1 and 2.

Equation 1

$$\frac{T_{Fusion}}{\sigma^2{}_{Fusion}} = \frac{T_{CBE}}{\sigma^2{}_{CBE}} + \frac{T_{ES}}{\sigma^2{}_{ES}}$$

Equation 2

$$\sigma^2{}_{Fusion} = \frac{\sigma^2{}_{CBE} \cdot \sigma^2{}_{ES}}{\sigma^2{}_{CBE} + \sigma^2{}_{ES}}$$

Here, T is temperature, and $\sigma^2$ is a distribution of temperature error. CBE refers to the CBE method, ES refers to the ES method, and Fusion refers to the CBE + ES method. Thus, $\sigma^2$Fusion refers to final error information, and TFusion refers to final temperature information.

As another example, when the merging unit 430 merges the candidate temperature images of the CBE image, the ES image, and the FS image, the merging unit 430 may perform the merging process using Equations 3 and 4.

Equation 3

$$\frac{T_{Fusion}}{\sigma^2_{Fusion}} = \frac{T_{CBE}}{\sigma^2_{CBE}} + \frac{T_{ES}}{\sigma^2_{ES}} + \frac{T_{FS}}{\sigma^2_{FS}}$$

Equation 4

$$\sigma^2_{Fusion} = \frac{\sigma^2_{CBE} \cdot \sigma^2_{ES} \cdot \sigma^2_{FS}}{\sigma^2_{CBE} \cdot \sigma^2_{ES} + \sigma^2_{CBE} \cdot \sigma^2_{FS} + \sigma^2_{ES} \cdot \sigma^2_{FS}}$$

Here, T is temperature, and $\sigma^2$ is a distribution of temperature error. CBE refers to the CBE method, ES refers to the ES method, FS refers to the FS method, and Fusion refers to the CBE + ES + FS method. Thus, $\sigma^2$Fusion refers to final error information, and TFusion refers to final temperature information.

The merging unit 430 may merge the candidate temperature images or the candidate error images using the equations above. However, the current embodiment is not limited to the equations above, and it will be apparent to one of ordinary skill in the art that any other method of respectively applying different weights to accuracies of the temperature determining methods may be used.

The final temperature image generating unit 4230 may generate a final temperature image based on the final temperature information merged by the merging unit 430. In addition, the final error image generating unit 4240 may generate the final error image based on final error information merged by the merging unit 430.

As a result, the final temperature image represents the result of merging the plurality of candidate temperature images obtained by different temperature determining methods, and the final error image represents the result of merging the plurality of candidate error images according to the accuracies of the different temperature determining methods.

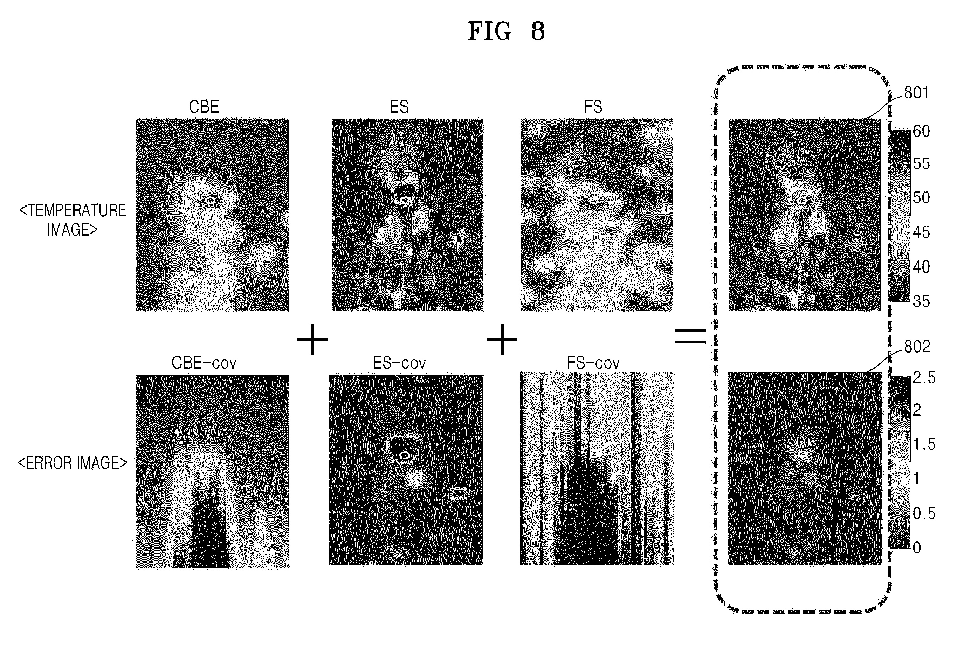

FIG. 8 shows a final temperature image and a final error image according to an embodiment of the present disclosure. FIG. 8 shows a final temperature image 801 (fusion image) produced by merging the CBE image, the ES image, and the FS image, which are candidate temperature images, and a final error image 802 (fusion-cov image) produced by merging the CBE-cov image, the ES-cov image, and the FS-cov image, which are candidate error images.

When the candidate error images are compared with the final error image, an overall error of the final error image is reduced compared with those of any individual one of the candidate error images. Accordingly, when the candidate temperature images are compared with the final temperature image, the tail artifact phenomenon of the CBE image is reduced in the final temperature image. In addition, while the ES image has low position accuracy of a focus region at high temperatures, the position accuracy of the focus is relatively high in the final temperature image. This is because only the results of the temperature determining methods with high accuracy are merged from the temperature determining methods.

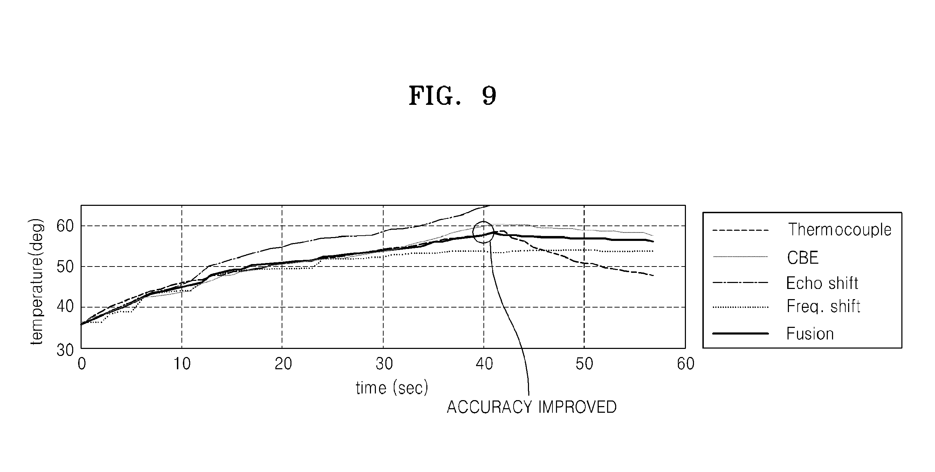

FIG. 9 is a graph illustrating a monitored temperature according to an embodiment of the present disclosure compared with a monitored temperature according to general methods. FIG. 9 shows temperature changes of the treatment site 610 (FIG. 1) while a treatment using therapeutic ultrasound is performed. When using each of the CBE method, the ES method, and the FS method alone, a temperature variation (thermocouple) cannot be accurately detected at a temperature ranging from about 50°C to about 60 °C where the treatment is completed. According to the current embodiment, however, the temperature variation (thermocouple) may be accurately detected by the Fusion method at a temperature ranging from about 36°C to about 58°C where the treatment begins and completed. Meanwhile, the graph of FIG. 9 only illustrates the results of simulations, and thus the current embodiment is not limited to the results of FIG. 9.

Referring back to FIG. 2, the display control unit 440 may control the display device 50 such that at least one of the candidate temperature images, the candidate error images, the final temperature image, and the final error image is displayed in the display device 50 (FIG. 1). That is, the display control unit 440 controls the display device 50 such that only an image which a user desires to display is displayed.

According to a user environment, the display control unit 440 may control the display device 50 such that only the temperature information of the treatment site 610 (FIG. 1) contained in the candidate temperature images or the final temperature image may be displayed. That is, for example, only information of the temperature variation according to the Fusion method shown in FIG. 9 may be displayed. Furthermore, the display control unit 440 may control the display device 50 such that only the error information of the treatment site 610 (FIG. 1) contained in the candidate error images or the final error image may be displayed.

As described above, the temperature monitoring device 40 according to the current embodiment provides the final temperature information produced by the merging of the results from different temperature determining methods, rather than using one temperature determining method, so that the temperature may be accurately monitored over a relatively wide range of temparatures.

Referring back to FIG. 1, the control device 30 controls operations and functions of the therapeutic ultrasound device 10 and the diagnostic ultrasound device 20.

According to another embodiment of the present disclosure, the control device 30 may control the therapeutic ultrasound device 10 such that the therapeutic ultrasound is automatically irradiated until the temperature of the treatment site 610 in the final temperature image generated by the temperature monitoring device 40 reaches a predetermined level.

That is, when the position of the treatment site 610 is recognized in the final temperature image, the temperature monitoring device 40 may monitor the temperature variation of the treatment site 610 in real time. Accordingly, the control device 30 may control the therapeutic ultrasound device 10 such that the irradiation of the therapeutic ultrasound is terminated when the temperature of the treatment site 610 reaches a predetermined level while continuously receiving the results of monitoring the temperature of the treatment site 610. In this regard, the predetermined level indicates a temperature at which the treatment has been completed.

The display device 50 includes a device that displays an image generated by the image generating unit 420, which image is received from the display control unit 440 (FIG. 2). The user may monitor temperature by identifying the temperature image or the error image displayed by the display device 50 while the ultrasound treatment is being performed. The display device 50 may include a device used for displaying visible information in order to report information to the user, for example, a general monitor, a LCD monitor, a LED monitor, and a scale-display device.

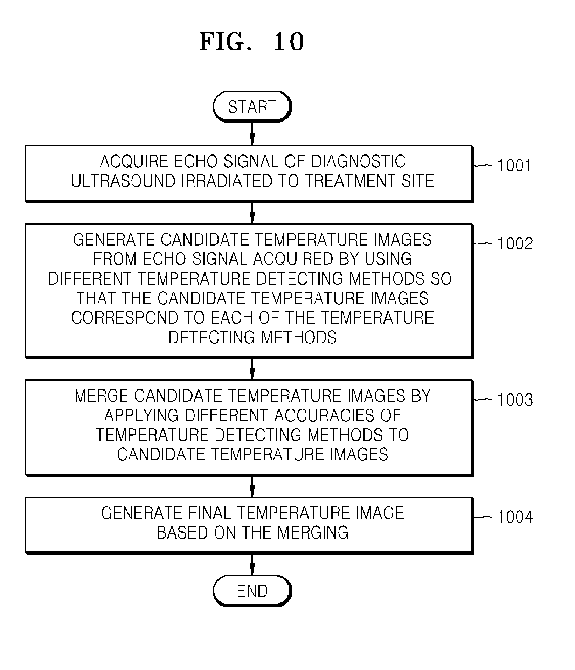

FIG. 10 is a flowchart of a method of monitoring temperature using ultrasound according to an embodiment of the present disclosure. Referring to FIG. 10, the method of monitoring temperature using ultrasound according to the current embodiment includes operations performed sequentially by the system for treatment and diagnosis 1 using ultrasound shown in FIG. 1 or the temperature monitoring device 40 shown in FIG. 2. Thus, descriptions given above in relation to FIGS. 1 and 2 may also be applied to the method of monitoring temperature using ultrasound and thus will not be provided here.

In operation 1001, an echo signal of diagnostic ultrasound irradiated to a treatment site is acquired, for example by the acquisition unit 410.

In operation 1002, candidate temperature images from the echo signal acquired using different temperature determining methods are generated so as to correspond to each of the temperature determining methods, for example by the candidate temperature image generating unit 4210.

In operation 1003, the candidate temperature images are merged by applying different accuracies of the temperature determining methods to the candidate temperature images, for example by the merging unit 430.

In operation 1004, a final temperature image based on the merging is generated, for example, by the final temperature image generating unit.

As described above, according to one or more of the above embodiments of the present disclosure, accurate final temperature information may be provided as a result of merging the results obtained from different temperature determining methods rather than using one temperature determining method in isolation. In particular, the temperature of the treatment site may be accurately monitored at high temperatures while the ultrasound treatment is performed, and thus ultrasound treatment may be efficiently performed, for example, a treatment time may be reduced. In addition, since the ultrasound treatment is performed using the temperature images of the treatment site and the peripheral areas thereof, normal tissues of the peripheral areas of the treatment site may be prevented from being damaged by the therapeutic ultrasound.

Meanwhile, embodiments of the present disclosure can be written as computer programs or program instructions and can be implemented in general-use digital computers that execute the programs using a non-transitory computer-readable recording medium. The computer readable code can be recorded/transferred on a medium in a variety of ways.

The media may also include, alone or in combination with the program instructions, data files, data structures, and the like. Examples of non-transitory computer-readable media include magnetic media such as hard disks, floppy disks, and magnetic tape; optical media such as CD ROM discs and DVDs; magneto-optical media such as optical discs; and hardware devices that are specially configured to store and perform program instructions, such as read-only memory (ROM), random access memory (RAM), flash memory, and the like.

Examples of program instructions include both machine code, such as produced by a compiler, and files containing higher level code that may be executed by the computer using an interpreter. The described hardware devices may be configured to act as one or more software modules in order to perform the operations of the above-described embodiments, or vice versa. Any one or more of the software modules described herein may be executed by a dedicated processor unique to that unit or by a processor common to one or more of the modules. The described methods may be executed on a general purpose computer or processor or may be executed on a particular machine such as the device described herein.

It should be understood that the exemplary embodiments described herein should be considered in a descriptive sense only and not for purposes of limitation. Descriptions of features or aspects within each embodiment should typically be considered as available for other similar features or aspects in other embodiments.

**Claims**

1. A device for monitoring temperature using ultrasound, the device comprising:

   an acquisition unit arranged to acquire an echo signal of diagnostic ultrasound irradiated to a treatment site;
   a candidate temperature image generating unit arranged to generate candidate temperature images from the acquired echo signal using different temperature determining methods, where each of the candidate temperature images corresponds to one of the respective temperature determining methods;
   a merging unit arranged to merge the generated candidate temperature images by combining portions of the generated candidate temperature images according to relative accuracies of the portions of the generated candidate temperature images; and
   a final temperature image generating unit arranged to generate and output a final temperature image based on the merged results.

2. The device of claim 1, wherein the temperature determining methods comprise methods of determining a temperature by detecting different types of waveform variations from the echo signal.

3. The device of claim 2, wherein the different types of waveform variations comprise at least two of echo time variation, amplitude variation, and shape variation of the echo signal.

4. The device of claim 1, 2 or 3, wherein the merging unit is arranged to merge temperature information represented by pixels respectively corresponding to a same position of the candidate temperature images.

5. The device of any preceding claim, wherein the merging unit is arranged to merge the generated candidate temperature images by respectively applying different weights based on the accuracies to temperature information of the generated candidate temperature images.

6. The device of any preceding claim, further comprising:

   at least an ultrasound device arranged to irradiate diagnostic ultrasound to the treatment site and to receive an echo signal of the diagnostic ultrasound;
   a temperature monitoring device arranged to acquire the received echo signal, to generate candidate temperature images from the echo signal by using different temperature determining methods where each of the candidate temperature images corresponds to one of the temperature determining methods, to merge the generated candidate temperature images by applying different accuracies of the temperature determining methods to the generated candidate temperature images, and to generate a final temperature image based on the merging; and
   a display device arranged to display at least one of the candidate temperature images and the final temperature image.

7. A method of monitoring temperature using ultrasound, the method comprising:

   acquiring an echo signal of diagnostic ultrasound that is irradiated to a treatment site;
   generating candidate temperature images from the echo signal using different temperature determining methods, where each of the candidate temperature images corresponds to one of the respective temperature determining methods;

merging the generated candidate temperature images by combining portions of the generated candidate temperature images according to relative accuracies of the portions of the generated candidate temperature images; and

generating and outputting a final temperature image based on the merging.

8. The method of claim 7, wherein the temperature determining methods comprise methods of determining a temperature by detecting different types of waveform variations in response to temperature variations of the treatment site from the echo signal.

9. The method of claim 8, wherein the different types of waveform variations comprise at least two of echo time variation, amplitude variation, and shape variation of the echo signal.

10. The method of any one or more than one of the preceding claims 7 - 9, wherein the temperature determining methods have accuracies that are different from each other at different temperature ranges.

11. The method of any one or more than one of the preceding claims 7 - 10, wherein the merging is performed by merging temperature information represented by pixels respectively corresponding to a same position of the candidate temperature images.

12. The method of any one or more than one of the preceding claims 7 - 11, wherein the merging is performed by applying different weights, based on the accuracies, to temperature information of the generated candidate temperature images.

13. The method of any one or more than one of the preceding claims 7 - 12, further comprising calculating errors of temperature information comprised in each of the generated candidate temperature images respectively based on accuracies of the temperature determining methods,

wherein the merging is performed by merging the generated candidate temperature images by calculating final temperature information by using the temperature information contained in each of the generated candidate temperature images and the calculated errors.

14. The method of any one or more than one of the preceding claims 7 - 13, further comprising generating candidate error images of the generated candidate temperature images which represent the calculated errors.

15. The method of claim 14, wherein the merging further comprises merging the generated candidate error images by calculating final error information by merging the error information contained in each of the generated candidate error images, and generating a final error image using the final error information.

# FIG. 1

CONTROL DEVICE 30

TEMPERATURE MONITORING DEVICE 40

DISPLAY DEVICE 50

# FIG. 2

40

**TEMPERATURE MONITORING DEVICE**

420

**IMAGE GENERATING UNIT**

410

ACQUISITION
UNIT

4210

CANDIDATE TEMPERATURE
IMAGE GENERATING UNIT

430

MERGING UNIT

4220

CANDIDATE ERROR
IMAGE GENERATING UNIT

440

DISPLAY
CONTROL
UNIT

4230

FINAL TEMPERATURE
IMAGE GENERATING UNIT

4240

FINAL ERROR IMAGE
GENERATING UNIT

# FIG. 3A

BEFORE HEATING

REFERENCE

AFTER HEATING

ECHO TIME VARIATION — 303

AMPLITUDE VARIATION — 304

SHAPE VARIATION — 305

301

302

# FIG. 3B

<ECHO SIGNAL>

ECHO SHIFT

BEFORE HEATING

SHAPE          AMPLITUDE

AFTER HEATING

306

# FIG. 4

## FIG. 5

### \<COMPARISON OF FOCAL POINT AT LOW TEMPERATURE\>

CBE ES CBE+ES

501

### \<COMPARISON OF FOCAL POINT AT HIGH TEMPERATURE\>

CBE ES CBE+ES

502

EP 2 559 375 A1

## FIG 6

ES

ES error (temperature)

ES error (NCC)

ES-cov 603

601

CBE

CBE error

ACCUMULATION OF DEPTH DIRECTION

CBE-cov 604

605

606

602

**FIG. 7**

# FIG 8

FIG. 9

# FIG. 10

START

ACQUIRE ECHO SIGNAL OF DIAGNOSTIC
ULTRASOUND IRRADIATED TO TREATMENT SITE — 1001

GENERATE CANDIDATE TEMPERATURE IMAGES
FROM ECHO SIGNAL ACQUIRED BY USING
DIFFERENT TEMPERATURE DETECTING METHODS SO
THAT THE CANDIDATE TEMPERATURE IMAGES
CORRESPOND TO EACH OF THE TEMPERATURE
DETECTING METHODS — 1002

MERGE CANDIDATE TEMPERATURE IMAGES BY
APPLYING DIFFERENT ACCURACIES OF
TEMPERATURE DETECTING METHODS TO
CANDIDATE TEMPERATURE IMAGES — 1003

GENERATE FINAL TEMPERATURE IMAGE
BASED ON THE MERGING — 1004

END

| | Europäisches Patentamt European Patent Office Office européen des brevets | **EUROPEAN SEARCH REPORT** | Application Number EP 12 18 0845 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JIGNESH SHAH,SUHYUN PARK,SALAVAT AGLYAMOV,TIMOTHY LARSON: "Photoacoustic imaging and temperature measurement for photothermal cancer therapy", SPIE, JOURNAL OF BIOMEDICAL OPTICS, vol. 13(3), 27 June 2008 (2008-06-27), pages 034024-1-034024-9, XP040446770, PO BOX 10 BELLINGHAM WA 98227-0010 USA | 1,4-7, 11-15 | INV. A61B5/01 G01K11/22 A61N7/02 A61B8/00 |
| Y | * abstract * * pages 034024-2, left-hand column, paragraph 1 - paragraph 3; figure figure 1 * * pages 034024-3, left-hand column, paragraph 1 - right-hand column, paragraph 1 * * pages 034024-3, right-hand column, paragraph 5 - pages 034024-7, right-hand column, paragraph 3; figures 2-8 * ----- | 2,3,8-10 | |
| Y | US 2011/087097 A1 (BEHAR BOAZ [IL]) 14 April 2011 (2011-04-14) * paragraphs [0008], [0086] * ----- -/-- | 2,3,8,9 | TECHNICAL FIELDS SEARCHED (IPC) A61B G01K A61N |

The present search report has been drawn up for all claims

| Place of search | Date of completion of the search | Examiner |
|---|---|---|
| The Hague | 16 November 2012 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 2 559 375 A1

Europäisches
Patentamt

European
Patent Office

Office européen
des brevets

# EUROPEAN SEARCH REPORT

Application Number

EP 12 18 0845

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HAO-LI LIU ET AL: "A unified approach to combine temperature estimation and elastography for thermal lesion determination in focused ultrasound thermal therapy;Combination of thermal imaging and elastography", PHYSICS IN MEDICINE AND BIOLOGY, INSTITUTE OF PHYSICS PUBLISHING, BRISTOL GB, vol. 56, no. 1, 9 December 2010 (2010-12-09), pages 169-186, XP020202345, ISSN: 0031-9155, DOI: 10.1088/0031-9155/56/1/011 * abstract * * page 179, paragraph 3 - page 180, paragraph 2; figure 11 * | 10 | |
| A | US 2009/105588 A1 (EMELIANOV STANISLAV [US] ET AL) 23 April 2009 (2009-04-23) * paragraphs [0012], [0014], [0041], [0096] - [0101] * | 2,3,8,9 | |
| A | SEIP R ET AL: "NONINVASIVE ESTIMATION OF TISSUE TEMPERATURE RESPONSE TO HEATING FIELDS USING DIAGNOSTIC ULTRASOUND", IEEE TRANSACTIONS ON BIOMEDICAL ENGINEERING, IEEE SERVICE CENTER, PISCATAWAY, NJ, USA, vol. 42, no. 8, 1 August 1995 (1995-08-01) , pages 828-839, XP000556811, ISSN: 0018-9294, DOI: 10.1109/10.398644 * abstract * | 2,3,8,9 | TECHNICAL FIELDS SEARCHED (IPC) |

-/--

The present search report has been drawn up for all claims

| Place of search | Date of completion of the search | Examiner |
|---|---|---|
| The Hague | 16 November 2012 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| | Europäisches Patentamt European Patent Office Office européen des brevets | EUROPEAN SEARCH REPORT | Application Number EP 12 18 0845 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | R. M. ARTHUR ET AL: "Non-invasive estimation of hyperthermia temperatures with ultrasound", INTERNATIONAL JOURNAL OF HYPERTHERMIA, vol. 21, no. 6, 1 January 2005 (2005-01-01), pages 589-600, XP055044612, ISSN: 0265-6736, DOI: 10.1080/02656730500159103 * abstract * ----- | 2,3,8,9 | |
| A | US 6 067 371 A (GOUGE JAMES [US] ET AL) 23 May 2000 (2000-05-23) * column 9, line 36 - column 11, line 29; figures 1,3a,3b,3c * ----- | 1-15 | |
| | | | TECHNICAL FIELDS SEARCHED (IPC) |

The present search report has been drawn up for all claims

| Place of search | Date of completion of the search | Examiner |
|---|---|---|
| The Hague | 16 November 2012 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 12 18 0845

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2012

| Patent document cited in search report | | Publication date | Patent family member(s) | | Publication date |
|---|---|---|---|---|---|
| US 2011087097 | A1 | 14-04-2011 | EP | 2519157 A1 | 07-11-2012 |
| | | | EP | 2519159 A1 | 07-11-2012 |
| | | | US | 2011087097 A1 | 14-04-2011 |
| | | | WO | 2011080712 A1 | 07-07-2011 |
| | | | WO | 2011080713 A1 | 07-07-2011 |
| US 2009105588 | A1 | 23-04-2009 | US | 2009105588 A1 | 23-04-2009 |
| | | | WO | 2009045885 A2 | 09-04-2009 |
| US 6067371 | A | 23-05-2000 | AU | 1062397 A | 19-06-1997 |
| | | | EP | 0864075 A1 | 16-09-1998 |
| | | | JP | 2000501530 A | 08-02-2000 |
| | | | US | 6067371 A | 23-05-2000 |
| | | | WO | 9720193 A1 | 05-06-1997 |

EPO FORM P0459

For more details about this annex : see Official Journal of the European Patent Office, No. 12/82